# EUROPÄISCHE PATENTSCHRIFT

(11) **EP 1 828 967 B1**
(45) Veröffentlichungstag und Bekanntmachung des Hinweises auf die Patenterteilung: **28.12.2016**
(21) Anmeldenummer: 05792078.7
(22) Anmeldetag: 23.08.2005
(51) Int. Cl.: G06M 1/16, G06M 1/22, G06M 1/04, A61M 15/00, B65D 83/14, G06M 1/08, G06M 1/24

(54) **INHALIERGERÄT**
INHALER
INHALATEUR

(30) Priorität: 10.11.2004 DE 102004054179; 18.07.2005 DE 102005033398
(43) Veröffentlichungstag der Anmeldung: 05.09.2007
(60) Teilanmeldung: 07116846.2
(73) Patentinhaber: Von Schuckmann, Alfred, 47627 Kevelaer (DE)
(72) Erfinder: Von Schuckmann, Alfred, 47627 Kevelaer (DE)
(74) Vertreter: Rieder, Hans-Joachim
(86) Internationale Anmeldenummer: PCT/EP2005/054138
(87) Internationale Veröffentlichungsnummer: WO 2006/051006

(56) Entgegenhaltungen:
- EP-A- 0 480 488
- EP-A- 1 065 477
- WO-A-01/28887
- WO-A-2004/071563
- US-A- 5 228 586
- US-A- 5 971 140
- US-A1- 2004 144 798
- US-A1- 2004 149 772
- US-A1- 2004 211 420
- US-B1- 6 431 168
- US-B1- 6 446 627

## Beschreibung

Die Erfindung betrifft ein Handgerät zur portionierten Ausgabe sprühfähiger Substanzen, insbesondere Inhaliermedikamenten, *gemäß Gattungsbegriff des Hauptanspritches.*

Handgeräte der in Rede stehenden Art *sind vorbekannt (*US 2004/149772*). Sie* finden insbesondere Anwendung in der medizinischen Aerosol-Therapie zur Behandlung von Erkrankungen der Atemwege. Dort ist ein Schrittschaltwerk vorgesehen, *dessen* Konstruktion hinsichtlich Plätzbedarf und Reinigungsmöglichkeiten nachteilig ist.

*Der Erfindung liegt deshalb die Aufgabe zugrunde,* ein Handgerät der in Rede stehenden Art in räumlich günstiger Weise bei vereinfachten Aufbau handhabungstechnisch sicherer auszugestalten.

Diese Problematik ist zunächst und im Wesentlichen durch den Gegenstand des Anspruches 1 gelöst, wobei auf ein in einem tellerförmigen Gehäuse angeordnetes Schrittschaltwerk mit Schaltgliedern abgestellt ist, dass *bei der Kartuschenbewegung das gesamte Gehäuse und damit dem Schrittschaltwerk ausgenommen aber ein Schrittschaltfinger-Stern mitbewegt wird.* Zufolge dieser Ausgestaltung ist ein Handgerät der in Rede stehenden Art geschaffen, welches bedingt durch die gewählte Konstruktion sowohl hinsichtlich des Platzbedarfes als auch hinsichtlich der Reinigungsmöglichkeiten und darüber hinaus auch hinsichtlich der Handhabbarkeit in vorteilhafter Weise ausgebildet ist. Es ist ein mechanisch arbeitendes Schrittschaltwerk *geschaffen,* welches aufgrund der gewählten Anordnung der verschiedenen Schaltglieder *insbesondere des Schrittschaltfinger-Sternes* eine günstige, raumsparende Bauform aufweist. *für die* da der unterhalb der Kartusche in dem Handgerätgehäuse vorhandene Freiraum genutzt werden kann. *Wird das Schrittschaltwerk zu Reinigungszwecken (mitsamt oder ohne Kartusche) entnommen, so ist die Gefahr einer Veränderung der Zählwerkstellung praktisch vermieden, weil der Schrittschaltfinger-Stern praktisch nur mit einem Manipulationswerkzeug zugänglich ist, bei anhängender Kartusche sogar nicht einrieal das.*

Die Gegenstände der weiteren Ansprüche sind nachstehend in Bezug zu dem Gegenstand des Anspruches 1 erläutert

So ist weiter vorgesehen, dass das Schrittschaltwerk einen *Skalenring* aufweist, der über ein Planetenrad-Getriebe von einem konzentrisch zum Ventilrohr umlaufenden, vom Öffnungshub der Kartusche angetriebenen Zahnkranz schrittweise gedreht wird. Die Drehbewegung des Skalenrings ist abgezweigt aus der *Verlagerung* der Kartusche durch das Schrittschaltwerk, dessen *Schrittschaltfinger-Stern* auf einer Bahn rund um das Kartuschen-Ventilrohr arbeitet. Der Schaltfinger-Stern ist Teil des integral aufgebauten Schrittschaltwerkes und findet Abstützung in der Betriebsstellung des Handgerätes im Bereich des gehäuseseitigen Ventilrohr-Aufnahmeabschnittes, welcher zugleich das Gegenlager für das Ventilrohr zur Ventilöffnung ausformt. Bei einer möglichen Entnahme des Schrittschaltwerkes aus dem Gehäuse ist wegen des dann fehlenden Stützabschnittes für den Schaltfinger eine versehentliche kartuschen-unabhängige Betätigung des Schrittschaltwerkes nicht möglich. Weiter ist vorgesehen, dass das Planetenrad in einer Bohrung des Skalenrings lagert und das zugehörige Sonnenrad auf einer unterseitig gezahnten Scheibe sitzt. Diese Scheibe steht in Eingriff mit dem Schaltfinger. Weiter greift in die Zahnung ein Rastfinger ein, zur Sicherung der jeweils erreichten Scheiben-Drehstellung. Sonnenrad und gezahnte Scheibe sind bevorzugt einteilig ausgeformt, dies bei koaxialer Ausrichtung. Weiter ist bevorzugt, dass die auf der äußeren Mantelfläche des Skalenrings angeordnete und vor einem Sichtfenster des Gehäuses laufende Skaleneinteilung des Skalenrings jeweils mehreren Einzel-Drehschritten des Planetenrades entspricht. Diesbezüglich erweist es sich weiterhin als vorteilhaft, dass hinsichtlich der Untersetzung ein Einzel-Drehschritt des Planetenrades nach Durchlauf mehrerer Einzel-Drehschritte des Sonnenrades erfolgt. Das Planetenrad steht weiter radial außen mit einem Zahnkranz in Eingriff. Letzterer weist mindestens einen von der unteren Randkante schräg aufwärts gerichtet ausgehenden Schlitz zum Eintritt des Schrittschaltfingers auf, um hierüber die Schaltrichtung des Fingers zum schrittweisen Weiterdrehen der sonnenradseitigen Scheibe vorzugeben. Der oder die Schrittschaltfinger sind schräg aufwärts von der Nabe ausgehend gerichtet, wobei weiter die Schrittschaltfinger sich beim Ausgabehub der Kartusche in Richtung einer Ebene senkrecht zur Längsachse bewegen. Nach einem Loslassen der Kartusche eilt diese aufgrund der integrierten Ventilfeder zurück in ihre Grundstellung, was durch die nunmehr fehlende Beaufschlagung des Schrittschaltwerkgehäuses ein Nacheilen desselben bewirkt. Dies ist durch die vorgespannten Schrittschaltfinger erreicht, die ihre ursprüngliche, schräg aufwärts gerichtete Position selbsttätig wieder einnehmen und hierbei die Schaltglieder sowie das Schrittschaltwerkgehäuse in ihre Ursprungslage drücken. Entsprechend ist das Schrittschaltwerk hinsichtlich der Rückverlagerung in eine Grundstellung entkoppelt von der Kartusche. *Das* als Flachteller gestaltete Schrittschaltwerkgehäuse *besitzt* ein zentrales Loch zum Durchtritt des Ventilrohrs welches Loch sich am kartuschenseitigen Ende zum Eintritt einer an der Kartuschen-Öffnungsseite vorstehenden Kragens verbreitert, hinter dessen Bereich eine federnde Verrastung zwischen Schrittschaltwerkgehäuse und Kartusche erfolgt. Die federnde Verrastung zwischen Schrittschaltwerkgehäuse und Kartusche kann so gewählt sein, dass diese nicht werkzeuglos aufhebbar ist. Die axiale Länge des gesamten Schrittschaltwerkgehäuses ist weiter bevorzugt angepasst an die Erstreckungslänge des Ventilrohres, demzufolge letzteres in der entnommenen Stellung aus dem Handgerätgehäuse von dem Schrittschaltwerkgehäuse weiter umfasst in einer geschützten Stellung verbleibt. Alternativ kann das als Flachteller gestaltete Schrittschaltwerkgehäuse im Handgerätgehäuse verrastet sein derart, dass es noch um den Öffnungshub einwärts verlagerbar ist. So kann das Schrittschaltwerkgehäuse bspw. bei einer Erstbestückung des Handgerätes zusammen mit der Kartusche in das Handgerätgehäuse eingeschoben werden, woraufhin das Schrittschaltwerkgehäuse abschließend eine in dem Gehäuse vorgesehene Rast hintergreift. In einer Weiterbildung des Erfindungsgegensta.ndes ist eine doppelte Verrastung des als Flachteller gestalteten Schrittschaltwerkgehäuses vorgesehen derart, dass neben der Verrastung am Kragen der Kartusche eine zweite stärkere Verrastung im Handgerätgehäuse erfolgt. Vor einer Erstbenutzung ist es häufig notwendig, das Handgerätgehäuse zunächst mit der Kartusche zu bestücken. Um dem Benutzer einen weiteren Arbeitsschritt zu ersparen, nämlich das Bestücken des Handgerätes mit dem Schrittschaltwerk, kann letzteres verrastet mit der Kartusche bereits vorliegen. Ein zu Reinigungszwecken hiernach folgendes Ausziehen der Kartusche aus dem Handgerätgehäuse führt zur Aufhebung der Verrastung zwischen Schrittschaltwerkgehäuse und Kartuschenkragen, demzufolge das Schrittschaltwerk in dem Handgerätgehäuse verbleibt. Diesbezüglich erweist es sich noch als vorteilhaft, wenn die stärkere Verrastung im Handgerätgehäuse nach Entfernung der Kartusche aus dem Handgerätgehäuse für sich auflösbar ist. In einer alternativen Ausgestaltung kann vorgesehen sein, dass die Kartusche durch Rückhaltefinger, die schräg abwärts weisend in dem Handgerätgehäuse ausgebildet sind, gegen Abziehen blockiert ist. Eine solche Lösung ist insbesondere dann von Vorteil, wenn zwischen dem Schrittschaltwerkgehäuse und der Kartusche keine Verbindung, insbesondere Rastverbindung vorgesehen ist.

Nachstehend ist die Erfindung anhand der beigefügten Zeichnung, welche lediglich verschiedene Ausführungsbeispiele darstellt, näher erläutert. Es zeigt:
- Fig. 1: in perspektivischer Explosionsdarstellung das Schrittschaltwerk für das erfindungsgemäße Handgerät;
- Fig. 2: einen Querschnitt durch das Schrittschaltwerk;
- Fig. 3: in einer Längsschnittdarstellung das Handgerät in einer ersten Ausführungsform, mit einem an einer Kartusche verrasteten, schematisch dargestellten Schrittschaltwerk;
- Fig. 4: eine der Fig. 3 entsprechende Schnittdarstellung, jedoch eine zweite Ausführungsform betreffend, bei welcher das schematisch dargestellte Schrittschaltwerk am Gehäuse verrastet ist;
- Fig. 4a: eine dritte Ausführungsform in einer Schnittdarstellung gemäß Fig. 4, bei welcher die Kartusche in dem Handgerätgehause gegen Abziehen blockiert ist;
- Fig. 5: in Längsschnittdarstellung das Handgerät in einer weiteren Ausführungsform, nach Trennen eines oberen Gehäuseteils von einem. Mundstück.

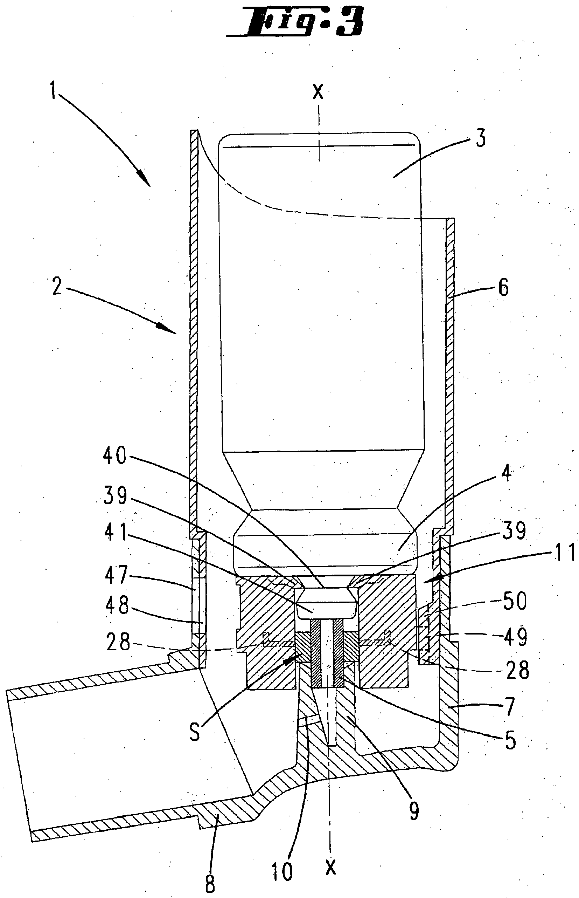

Das in Fig. 3 in einer schematischen Sclinittdärstellüng gezeigte Handgerät 1 dient zur portionierten Ausgabe sprühfähiger Substanzen, insbesondere von Inhaliermedikamenten.

Hierzu weist das Handgerät 1 zunächst ein Handgerätgehäuse 2 auf, in welches eine die sprühfähige Substanz beinhaltende Kartusche 3 einsetzbar ist. Diese Kartusche 3 ist in dem Gehäuse 2 axial verschiebbar.

In üblicher Weise weist der Kartuschenkopf 4 ein zentrales, sich koaxial zur Kartusche 3 erstreckendes Ventilrohr 5 auf. Über letzteres wird eine Medikamentenausgabe durch eine axiale Relativbewegung zwischen Kartusche 3 und Gehäuse 2 erreicht.

Das Gehäuse 2 ist zweigeteilt und besteht im Wesentlichen aus zwei übereinander angeordneten Ringteilen 6 und 7, von welchen das obere Ringteil 6 schaftartig ausgeformt ist und das untere Ringteil 7 ein etwa quer zur Schäfterstreckung ausgerichtetes Mundstück 8 aufweist. Letzteres ist durch eine nicht dargestellte Abdeckkappe verschließbar.

Das Ventilrohr 5 der Kartusche 3 stützt sich in einem zugeordneten rohrförmigen Stützabschnitt 9 innerhalb des unteren Ringteiles 7 ab, dies bei axialer Beweglichkeit der Kartusche 3 innerhalb des die Kartusche 3 umgebenden, schaftartigen Ringteils 6.

Der das Ventilrohr 5 der Kartusche 3 klemmend aufnehmende, innerhalb des unteren Gehäuse-Ringteils 7 ausgeformte Stützabschnitt 9 ist mit einem gegenüber einen das Ventilrohrende aufnehmenden Abschnitt durchmesserverringerten Strömungskanal 10 versehen, welcher strömungstechnisch in Verbindung steht mit dem Ventilrohr 5, wobei das dem Ventilrohr 5 abgewandte Ende des Strömungskanals 10 in Richtung auf das Mundstück 8 weist.

Die beiden Ringteile 6 und 7 sind in dem dargestellten Ausführungsbeispiel steckbar miteinander verbunden. Alternativ können die beiden Teile auch über ein Gewinde, bspw. über ein Gröbgewinde mit hoher Steigung, miteinander verbunden sein.

Die Anordnung der Kartusche 3 in dem Gehäuse 2 ist so gewählt, dass der Kartuschenkopf 4 in dem Gehäuse 2 etwa auf Höhe des Verbindungsbereiches zwischen Ringteil 6 und Ringteil 7 platziert ist.

Zentral unterhalb der öffnungsseitigen Stirnwand der Kartusche 3 ist in Überlappung zum Kartuschen-Ventilrohr 5 ein Schrittschaltwerk 11 angeordnet. Dieses dient zum Registrieren und Anzeigen der durchgeführten Ausgabebetätigungen, dies in Abhängigkeit von den durchgeführten Öffnungshüben der Kartusche 3.

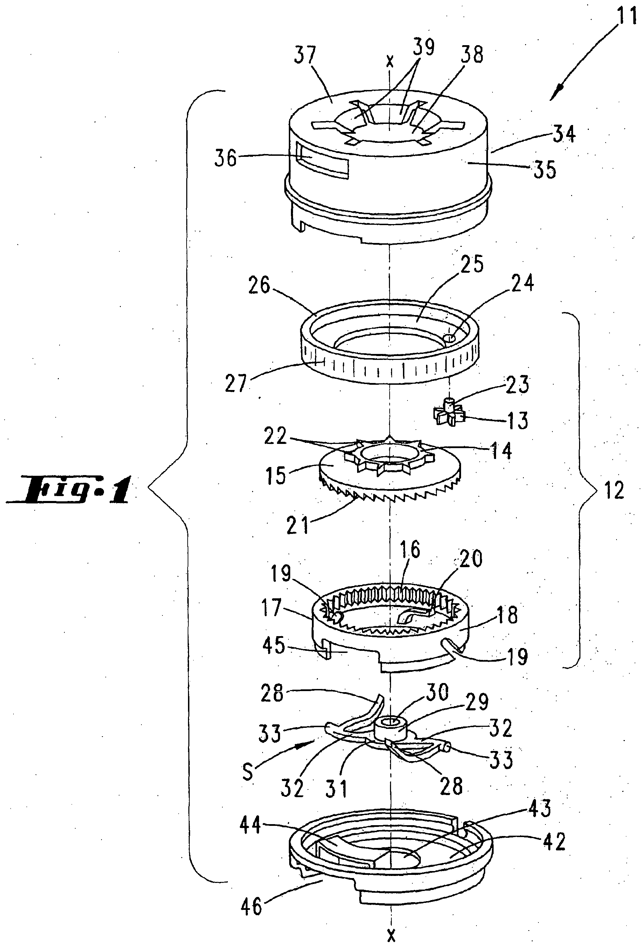

Das Schrittschaltwerk 11 ist in Fig. 1 in einer perspektivischen Explosionsdarstellung gezeigt. Zentraler Bestandteil des Schrittschaltwerks 11 ist ein Planetenrad-Getriebe 12, bestehend aus einem Planetenrad 13, einem Sonnenrad 14, welches auf einer unterseitig gezahnten Scheibe 15 sitzt und einem mit dem Planetenrad 13 zusammenwirkenden Zahnkranz 16. Letzterer ist wandungsinnenseitig eines nicht drehbar gehalterten, rohrabschnittförmigen Ringes 17 ausgeformt. Die Mantelwandung 18 des Ringes 17 ist in diametral gegenüberliegenden Bereichen durchsetzt von schräg aufwärts in Schaltrichtung gerichtet ausgehenden Schlitzen 19, die nach unten zur dem Zahnkranz 16 abgewandten Ringkante hin offen auslaufen.

Der Zahnkranz 16 erstreckt sich in axialer Richtung etwa über die halbe Höhe des Ringes 17, dessen Mantelwandung 18 zur dem Zahnkranz 16 abgewandten Ringstirnkante hin abgestuft, sich radial verjüngend ausgebildet ist.

Unterhalb des Zahnkranzes 16 ist innenseitig der Mantelwandung 18 des Ringes 17 ein Rastfinger 20 angeformt. Dieser ist mit Bezug auf einen Grundriss des Ringes gegenüber dem Zahnkranz 16 nach radial innen versetzt; greift entsprechend in einen zum Zahnkranz 16 nach radial innen eingezogenen Kreisraum. Weiter ist die Anordnung des in etwa in Vertikalrichtung elastisch ausgebildeten Rastfingers 20 so gewählt, dass dieser etwa in eine durch die unteren Randkanten des Zahnkranzes 16 aufgespannte Horizontalebene eingreift.

Der Durchmesser der das Sonnenrad 14 tragenden Scheibe 15 ist geringfügig kleiner gewählt, als der Innendurchmesser des Ringes 17 im Bereich des Zahnkranzes 16. Sonnenrad 14 und Scheibe 15 sind bevorzugt einstückig, materialeinheitlich ausgebildet.

Unterseitig der Scheibe 15 ist eine randbezogen umlaufende Sägezahnung 21 vorgesehen, in welche der Rastfinger 20 des Ringes 17 eingreift.

Das Sonnenrad 14 weist eine grobe Verzahnung auf. So sind in dem dargestellten Ausführungsbeispiel über den Umfang des Sonnenrades 14 acht Sonnenrad-Zähne 22 gleichmäßig verteilt ausgeformt. Diese Zähne 22 wirken im Zuge der Sonnenrad-Umdrehung mit dem in selber Ebene zwischen dem Sonnenrad 14 und dem Zahnkranz 16 des Ringes 17 angeordneten Planetenrad 13 zusammen.

Das Planetenrad 13 besitzt einen einseitig nach oben, d. h. von der Scheibe 15 des Sonnenrades 14 weg abragenden Achszapfen 23. Dieser ist in einer Bohrung 24 im Bereich eines scheibenartig nach radial innen weisenden Kragens 25 eines Skalenringes 26 drehbar gefasst. Der Skalenring 26 ist mantelaußenwandig mit einer umlaufenden Skaleneinteilung 27 versehen, wobei die Skaleneinteilung jeweils mehreren Einzel-Drehschritten des den Skalenring 26 weiterführenden Planetenrades 13 entspricht.

Die schrittweise Verlagerung des Sonnenrades 14 bzw. der einstückig hiermit ausgeformten Scheibe 15 erfolgt über etwa vertikal federnd ausweichbar ausgebildete Schrittschaltfinger 28. Diese greifen unterseitig in die Sägezahnung 21 der Scheibe 15.

Die Schrittschaltfinger 28 sind mit Bezug zu der Hauptachse x des gesamten Schrittschaltwerkes 11 diametral gegenüberliegend angeordnet. Hierzu ist zunächst ein zylinderförmiger Zentralkörper in Form einer Nabe 29 vorgesehen mit einer zentralen axialen Durchgangsbohrung 30. Deren Durchmesser ist geringfügig größer bemessen als der Außendurchmesser des diese Durchgangsbohrung 30 zu durchsetzenden Kartuschen-Ventilrohres 5.

Fußseitig geht die Nabe 29 über in einen radial erweiterten Kragen 31. An diesem sind diametral gegenüberliegend in Radialrichtung abragende Führungsabschnitte 32 angeformt, die jeweils im Bereich ihrer freien Enden einen in dem zugeordneten Schlitz 19 des Ringes 17 einliegende Führungszapfen 33 ausformen.

Die Schrittschaltfiriger 28 wurzeln jeweils mit einem Horizontalabschnitt an den Führungsabschnitten 32 unter Belassen der über den horizontalen Abschnitt radial außen abragenden Führungszapfen 33. Die von den Horizontalabschnitten abragenden Schrittschaltfinger 28 gehen schräg aufwärts gerichtet aus, etwa unter Einschließen eines Winkels von 45 Grad zur Horizontalen, angepasst an die Schrägung der Schlitze 19 im Ring 17. Der so gebildete Schrittschaltfinger-Stern trägt das Bezugszeichen S.

Der Schrittschaltfinger-Stern S, der den inneren Zahnkranz 16 aufweisende Ring 17, die einstückig mit dem Sonnenrad 14 ausgeformte Scheibe 15 sowie der Skalenring 26 sind konzentrisch zueinander auf der Achse x ausgerichtet, wobei die Höhe des Ringes 17 so gewählt ist, dass sowohl der Schrittschaltfinger-Stern S als auch das Sonnenrad 14 samt Scheibe 15 in diesem aufgenommen sind.

Das gesamte Planeten-Getriebe 12, sowie der Schrittschaltfinger-Stern S und der Skalenring 26 sind aufgenommen in einem topfartigen Schrittschaltwerkgehäuse 34 mit einem Außendurchmesser, welcher an den Außendurchmesser der Kartusche 3 angepasst ist.

Das Gehäuse 34 besitzt eine Mantelwandung 35. Diese weist ein Sichtfenster 36 auf, durch welches die Skaleneinteilung 27 des Skalenrings 26 erkennbar ist.

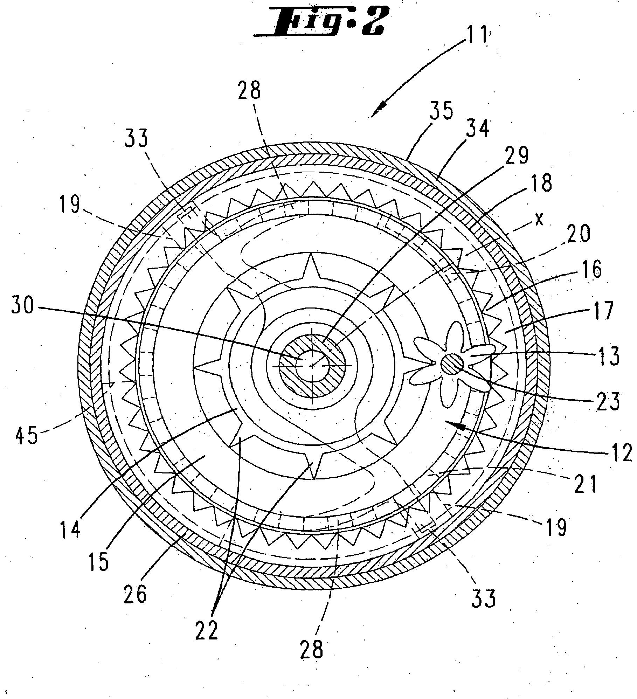

Die Gehäusedecke 37 besitzt eine zentrale Durchbrechung 38, welche in der in den Figuren 1 bis 3 gezeigten Ausführungsform umfasst ist von konisch zum Gehäuseinnern hin zulaufenden Rast-Federzungen in der in den Figuren 1 bis 3 gezeigten Ausführungsform 39. Der Durchbrechungs-Durchtnesser ist angepasst an einen Durchmesser eines Taillenabschnittes 40 eines über die öffnungsseitige Stirnwand der Kartusche 3 zentral überragenden Kragens 41, aus welch letzterem das Ventilrohr 5 auswächst.

Der Gehäuseboden 42 ist gebildet durch ein gesondertes Teil. Dieses ist unter Aufnahme der vorbeschriebenen Schrittschaltwerk-Einzelteile mit dem Gehäuse 34 verbunden, so bspw. mit diesem verschweißt oder über eine Presspassung an diesem gehaltert.

Der tellerartige Gehäuseboden 42 besitzt eine zentrale Bohrung zum Durchtritt des Ventilrohres 5. Des Weiteren ist auf dem Gehäuseboden 42 ein Reststück 44 ausgeformt, welches zur lageorientierten Fesselung des Ringes 17 in eine entsprechend in dessen Mantelwandung 18 ausgeformte, fensterartige Ausnehmung 45 greift.

Im selben Winkelbereich, in welchem das Passstück 44 auf dem Boden angeordnet ist, weist die Mantelaußenwandung des Gehäusebodens 42 einen Freischnitt 46 auf. Diese ist im Einbauzustand dem Bereich des Ausgangsquerschnittes des Strömungskanals 10 im unteren Ringteilgehäuse 7 zugeordnet.

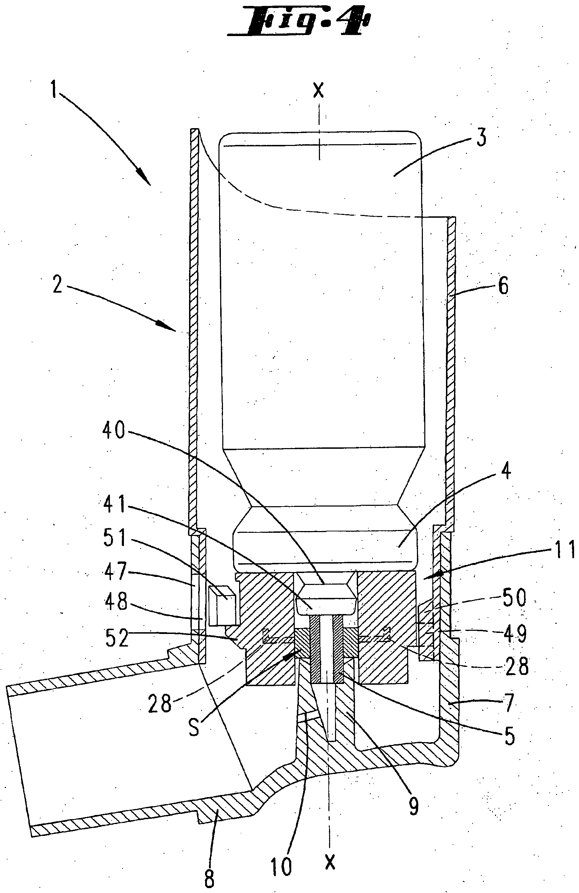

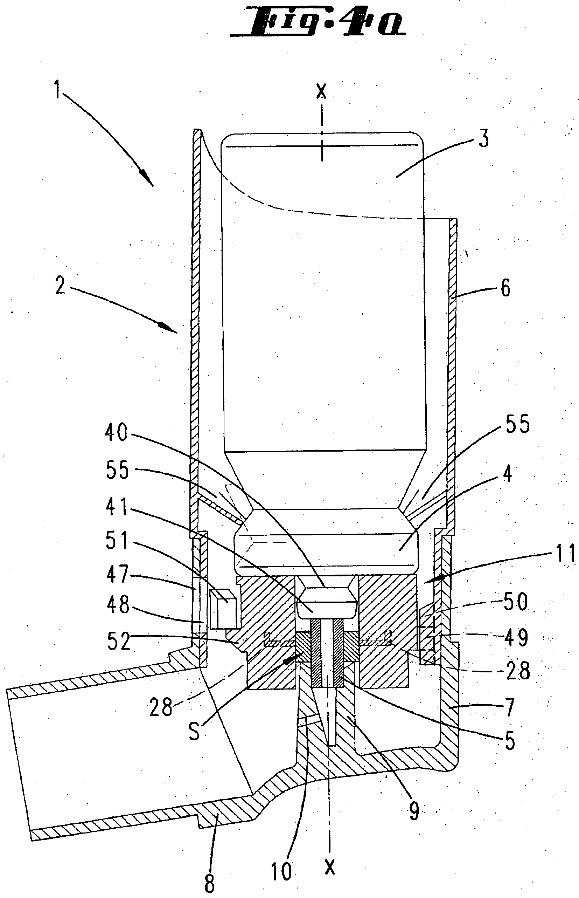

Die Funktionsweise des Schrittschaltwerkes 11 ist unäbhängig von der anhand der Figuren 3 und 4 noch zu beschreibenden grundsätzlichen Anordnung wie folgt:

Die Schaltglieder (Schrittschaltfinger-Stern S, Ring 17, Scheibe 15, Planetenrad 13 und Skalenring 26) wie auch das Gehäuse 34 mit dem Gehäuseboden 42 sind auf Achsen angeordnet, die sich in Längsrichtung der Kartusche 3 erstrecken. So sind mit Ausnahme des Planetenrades 13 alle weiteren Bauteile des Schrittschaltwerkes 11 auf der Kartuschenlängsachse x - x positioniert.

Das Schrittschaltwerk 11 ist entsprechend konzentrisch zum Ventilrohr 5 im Schatten der Kartusche 3 innerhalb des Gehäuses 2 angeordnet, dies konkret in dem zwischen Kartuschenkopf 4 und Stützabschnitt 9 des Gehäuses 2 belassenen Bauraum. Das Schrittschaltwerk 11 stützt sich mit der in dem Schaltwerkgehäuse 34 zentral gelagerten Nabe 29 des Schrittschaltfinger-Sterns S auf der Stirnfläche des Stützabschnitts 9 des Inhaliergehäuses 2 ab. Die zentrale Achse x des Schnittschaltwerks 11 wird von der Körperachse der Kartusche 3 übernommen. Das die Nabe 29 durchsetzende Ventilrohr 5 bietet eine zusätzliche Zentrierung der gesamten Schrittschaltwerk-Einheit.

Bei Durchführung eines Betätigungshubs der Kartusche 3 und damit einhergehender Vertikalverlagerung derselben in Richtung auf den Stützabschnitt 9 wird das Schaltwerkgehäuse 34 über den Kartuschenkopf 4 mitgeschleppt, dies unter Relativverlagerung des Gehäuses 34, des Planetenrad-Getriebes 12 und des Skalenringes 26 zu dem Schrittschaltfinger-Stern S, welche eine Abstützung auf dem Stützabschnitt 9 erfährt. Infolgedessen bewirken die sich spannenden Schrittschaltfinger 28 weiter unterstützt durch Drehaufgleiten des Schrittschaltfinger-Sterns S in den mantelwandseitigen Schlitzen 19 des Ringes 17 einen schrittweisen Drehvorschub der sägeverzahnten Scheibe 15. Einhergehend damit dreht sich das Sonnenrad 14 um denselben Winkelbetrag. Die Schrittschaltfinger 28 bewegen sich hierbei aus der schrägen Ausrichtung in Richtung auf eine senkrecht zur Längsachse x - x ausgerichtete Ebene.

Dadurch bedingt, dass das Sonnenrad 14 lediglich acht gleichmäßig über den Umfang verteilt angeordnete Zähne aufweist, führt nicht jede Schritt-Drehbewegung des Sonnenrades 14 zwangsläufig zu einer Drehbewegung des Planetenrades 13. Vielmehr wird die Drehung des Planetenrades 13 um dessen Achse und eine die damit einhergehende Drehverlagerung des Skalenringes 26 erst nach mehreren Einzel-Drehschritten des Sonnenrades 14 durchgeführt.

Gemäß der Darstellung in Fig. 3 ist das gesamte Schrittschaltwerk 11 mittels des Gehäuses 34 an dem kartuschenkopfseitig vorragenden Kragen 41 rastend festlegbar. Zugeordnet zu dem gehäuseseitigen Sichtfenster 36 weisen die zugeordneten Abschnitte der Gehäuseringteile 6 und 7 gleichfalls Sichtfenster 47, 48 auf, welche durch die gewählte Position, zugewandt dem Mundstück 8 des Gehäuses 2, im Blickfeld des das Handgerät 1 bedienenden Benutzers liegen. Zur lageorientierten Einführung des Schrittschaltwerkes 11 ist dieses mit einem radial von dem Gehäuse 34 abragenden Führungsblatt 49 versehen, welches in eine nicht näher dargestellte Vertikalnut im Innern des Gehäuses 2, den Verschiebeweg bei Hubbetätigung zulassend eingreift.

Die Verrastung zwischen Schrittschaltwerk 11 und Kartusche 3 im Bereich des kartuschenkopfseitigen Kragens 41 ist so gewählt, dass bei einer Entnahme der Kartusche 3 aus dem Gehäuse 2 das Schrittschaltwerk 11 an der Kartusche 3 verbleibend mit ausgezogen wird.

Alternativ besteht auch die Möglichkeit, wie in Fig. 4 schematisch dargestellt, dass das Schrittschaltwerk 11 mit dem Gehäuse 2 verrastet ist. Hierzu überfährt das Schrittschaltwerk 11 im Zuge einer Erstbestückung einen oder mehrere radial nach innen einragende Rastvorsprünge 51 des Gehäuses 2, welche Rastvorsprünge 51 hiernach von radial von dem Schaltwerkgehäuse 34 abragenden Kragenabschnitten 52 unterfangen sind.

Die Rastvorsprünge 51 sind in ihrer Vertikalausrichtung so positioniert, dass die Vertikalverlagerbarkeit des Schrittschaltwerkes 11 bei Hubbetätigung der Kartusche 3 gewährleistet ist.

Durch die gewählte Anordnung formen die Rastvorsprünge 51 Niederhalten aus, welche das Schrittschaltwerk 11 bei einem Abziehen der Kartusche 3 in dem Gehäuse 2 zurückhalten.

In einer weiteren, nicht dargestellten Ausführungsform kann eine Kombination der Ausführungsformen gemäß den Darstellungen in den Figuren 3 und 4 vorgesehen sein, bei welcher das Schrittschaltwerk 11 mittels der Rast-Federzunge 39 an dem kartuschenkopfseitigen Kragen 41 rastfestgelegt ist. Eine solche vormontierte Kartuschen-Schrittschaltwerk-Einheit wird vor einer Erstbenutzung des Handgeräts 1 in das Gehäuse 2 eingeführt, wobei an dem SchaltwerkGehäuse 34 radial abragende Kragenabschnitte 52 gerätegehäuseseitige Rastvorsprünge 51 entsprechend dem zweiten Ausführtmgsbeispiel überlaufen, was zu einer endgültigen Festlegung des Schrittschaltwerkes 11 in dem Gehäuse 2 führt. Diese Verrastung zwischen Schrittschaltwerk 11 und Gehäuse 2 ist stärker gewählt als die Verrastung zwischen Schrittschaltwerk 11 und Kartusche 3, demzufolge ein Abziehen der Kartusche 3 nach einer Erstbenutzung zu einer Rastaufhebung zwischen Kartusche 3 und Schrittschaltwerk 11 führt. Das Wiedereinsetzen der Kartusche 3 ist durch eine relativ schwache Rastfederausformung zur Zusammenwirkung mit dem kartuschenkopfseitigen Kragen 41 erleichtert.

Sowohl bei der vorbeschriebenen Ausführungsform als auch bei der Ausführungsform gemäß der Darstellung in Fig. 4 besteht die Möglichkeit, die Verrastung zwischen Gerätegehäuse 2 und Schrittschaltwerk 11 nach Entfernen der Kartusche 3 aus dem Gehäuse 2 für sich aufzulösen.

Die Darstellung in Fig. 4a zeigt eine weitere Ausführungsform, bauend auf der in Fig. 4 gezeigten Ausgestaltung. So ist zur weiteren Festlegung der Kartusche 3 - neben einer üblichen Klemmhalterung des Ventilrohrs 5 in dem handgerätgehäuseseitigen Stützabschnitt 9 - eine Blockierung der Kartusche 3 im Bereich des oberen Ringteil-Gehäuses 6 vorgesehen. So ragen mantelinnenseitig dieses oberen Gehäuseringteiles 6 schräg abwärts gerichtete Rückhaltefinger 55 ab, welche materialeinheitlich, einstückig mit dem oberen Gehäuseteil 6 gebildet sind. Diese Rückhaltefinger 55 sind so positioniert, dass deren freien Randkanten in der Zuordnungsstellung zur Kartusche 3 in den hinter dem Kartuschenkopf 4 ausgeformten Taillenbereich der Kartusche 3 sperrend eintreten, um so die Kartusche 3 zu blockieren.

Weiter sind die Rückhaltefinger 55 derart ausgeformt, dass diese zumindest bei einer Erstbestückung des Gehäuses 2 mit der Kartusche 3 durch den Kartuschenkopf 4 überlaufen werden können.

Die Kartusche 3 ist somit an dem Handgerätgehäuse 2, insbesondere an dem oberen Gehäuseteil 6 gefesselt.

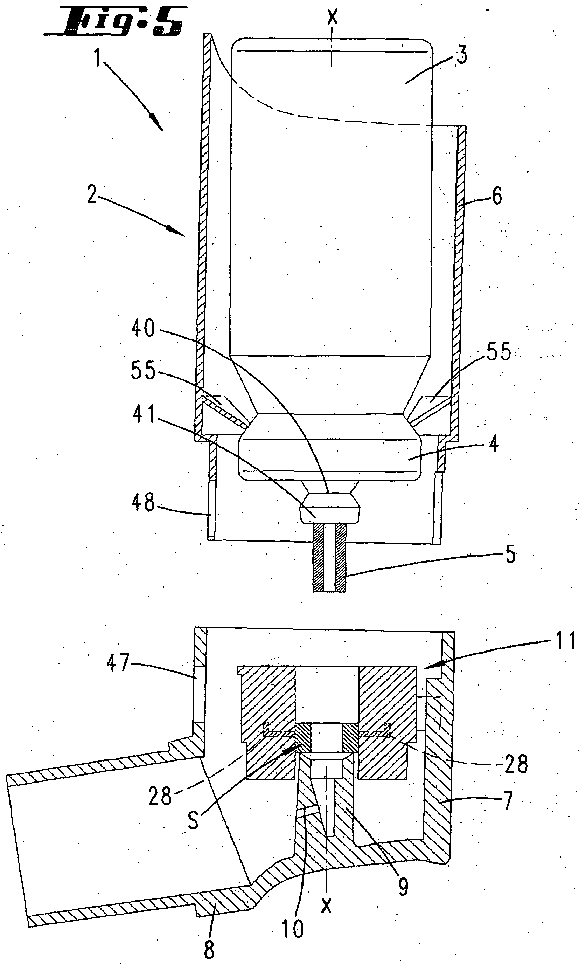

Die Darstellung in Fig. 5 zeigt eine weitere Ausführungsform. In dieser ist auch die Kartusche 3 mittels Rückhaltefingern 55 an dem oberen Gehäuseteil 6 gehaltert. Dieses obere Gehäuseteil 6 ist von dem unteren Gehäuseteil 7, welches das Mundstück 8 ausformt, lösbar, wobei die Trennung der beiden Gehäuseteile 6 und 7 etwa im Bereich der Position des tellerförmigen Schrittschaltwerkgehäuses 34 vorgenommen wird.

In der zusammengesetzten Stellung der beiden Gehäuseteile 6 und 7 sind diese bevorzugt verrastet, wozu ein Gehäuseteil eine Rastnase und das andere Gehäuseteil eine entsprechend platzierte Rastaufnahme aufweist.

Zufolge dieser ermöglichten Trennung ist eine verbesserte Reinigung des Mundstückes 8, insbesondere des den Stufenabschnitt 9 aufweisenden Knickabschnittes erreicht. Dies ist weiter noch dadurch erleichtert, dass das gesamte Schrittschältwerk 11, welches als kompakte Baugruppe geformt ist, in einfachster Weise aus dem unteren Gehäuseteil 7 entnehmbar ist und so einer gesonderten Reinigung zugeführt werden kann. In dem dargestellten Ausführungsbeispiel sind keine Festlegungsmittel - wie bspw. Rastvorsprünge 51, welche mit Kragenabschnitten 52 zusammenwirken, vorgesehen. Vielmehr ist eine Festlegung des gesamten Schrittschaltwerks 11 in dem unteren Gehäuseteil 7 in Zusammenwirkung mit der Kartusche 3 in der Gebrauchsstellung erreicht, dies unter Ausrichtung des Schrittschaltwerks 11 zwischen dem Stützabschnitt 9 und der zugewandten Stirnfläche des Kaituschenköpfes 4 (wie auch anhand der in Fig. 4a gezeigten Ausführungsform dargestellt).

Auch in dieser Ausführungsform ist das Schrittschaltwerk 11 gegen Drehverlagerung um die Achse x - x durch einen Formschluss zwischen Schrittschaltwerk 11 und unterem Gehäuseteil 7 gesichert.

## Patentansprüche

1. Handgerät (1) zur portionierten Ausgabe sprühfähiger Substanzen, insbesondere Inhaliermedikamenten, mit einer durch Drücken in einem Gehäuse (2) in die Ausgabe-Öffnungs-Stellung *linear* verschiebbaren, *mit ihrem Ventilrohr (5) an einem Stützabschnitt (9) aufsitzenden* Kartusche (3) und einem beim Öffnungshub der Kartusche (3) bewegten Schaltwerk (11) zum Registrieren und Anzeigen durchgeführter Ausgabebetätigungen, welches Schaltwerk (11) in einem Gehäuse (34) zentral unterhalb der öffnungsseitigen Stirnwand der Kartusche (3) überlappend zum Kartuschen-Ventilrohr (5) angeordnet ist *und mit im* Gehäuse (34) *angeordneten Schrittschaltwerk-Schaltgliedern* (S, 28, 29,16,17, 20,15, 21, 22,13, 26), die um in Längsrichtung der Kartusche (3) liegende Achsen umlaufen, *wobei federnde Schrittschaltfinger (28) sich in Richtung einer Querlage zur Längsrichtung verbiegen und eine Drehung des Schrittschaltwerkes veranlassen, **dadurch gekennzeichnet, dass** das Gehäuse (34) einschließlich seines Bodens (42) und des Schrittschaltzverkes (12, 12) als Ganzes, - mit Ausnahme eines auf dem Stützabschnitt (9) aufliegenden Schrittschaltfinger-Sternes (S), - vom Vorschub der Kartusche relativ zum Stützabschnitt (9) abwärts verlagerbar ist und die Verlagerungsbezuegung den Schrittschaltfinger-Stern (S) dreht.*

2. Handgerät nach Anspruch 1, **dadurch gekennzeichnet, dass** die Schrittschaltwerk-Schaltglieder einen Zahnkranz (16) umfassen, der mindestens einen von der unteren Randkante schräg aufwärts gerichtet ausgehenden Schlitz (19) besitzt zum Eintritt von mindestens einem Führungszapfen (33) des Schrittschaltfinger-Sternes (S).

3. Handgerät nach einem oder mehreren der vorhergehenden Ansprüche, **dadurch gekennzeichnet, dass** das als Flachteller gestaltete Schrittschaltwerkgehäuse (34) ein zentrales Loch (38) zum Durchtritt des Ventilrohrs (5) besitzt, welches Loch (38) sich am kartuschenseitigen Ende zum Eintritt einer an der Kartuschen-Öffnungsseite vorstehenden Kragens (41) verbreitert, hinter dessen Bereich eine federnde Verrastung zwischen Schrittschaltwerkgehäuse (34) und Kartusche (3) erfolgt.

4. Handgerät nach einem oder mehreren der vorhergehenden Ansprüche, **gekennzeichnet durch** eine doppelte Verrastung des als Flachteller gestalteten Schrittschaltwerkgehäuses (34) derart, dass neben der Verrastung am Kragen (41) der Kartusche (3) eine zweite stärkere Verrastung im Handgerätegehäuse (2) erfolgt.

5. Handgerät nach Anspruch 4, **dadurch gekennzeichnet, dass** die stärkere Verrastung im Handgerätegehäuse (2) nach Entfernung der Kartusche (3) aus dem Handgerätegehäuse (2) für sich auflösbar ist.

6. Handgerät nach einem oder mehreren der vorhandenen Ansprüche, **dadurch gekennzeichnet, dass** die Kartusche (3) durch Rückhaltefinger (55), die schräg abwärts weisend in dem Handgerätegehäuse (2) ausgebildet sind, gegen Abziehen blockiert ist.

## Claims

1. Hand-held device (1) for apportioned delivery of sprayable substances, in particular inhaler medicaments, having a cartridge (3) which has its valve tube (5) seated on a supporting portion (9) and is linearly displaceable by pressing in a housing (2) into the open position for delivery, and an indexing mechanism (11) which is moved during the opening stroke of the cartridge (3) for registering and displaying delivery actuations which have been carried out, which indexing mechanism (11) is disposed in a housing (34) centrally below the opening-side end wall of the cartridge (3), overlappingly with respect to the valve tube (5) of the cartridge, and having, disposed in the housing (34), step-by-step indexing mechanism indexing members (S, 28, 29, 16, 17, 20, 15, 21, 22, 13, 26) which revolve about axes lying in the longitudinal direction of the cartridge (3), resilient step-by-step indexing fingers (28) bending in the direction of a position transverse to the longitudinal direction and effecting a rotation of the step-by-step indexing mechanism, **characterized in that** the housing (34), including its base (42) and the step-by-step indexing mechanism (11, 12) - except for a step-by-step indexing finger star (S) located on the supporting portion (9) - is displaceable downward as a whole relative to the supporting portion (9) by the advance of the cartridge and the displacement movement rotates the step-by-step indexing finger star (S).

2. Hand-held device according to claim 1, **characterized in that** the step-by-step indexing mechanism indexing members include a toothed ring (16), which has at least one slot (19), which extends from the lower peripheral edge in a direction obliquely upwards, for entry of at least one guide pin (33) of the step-by-step indexing-finger star (S).

3. Hand-held device according to one or more of the preceding claims, **characterized in that** the step-by-step indexing-mechanism housing (34), which is configured as a flat plate, has a central hole (38) for the passage of the valve tube (5), which hole (38) widens at the cartridge end for the entry of a collar (41) which protrudes on the opening side of the cartridge and behind the region of which a resilient latching between step-by-step indexing-mechanism housing (34) and cartridge (3) takes place.

4. Hand-held device according to one or more of the preceding claims, **characterized by** a double latching of the step-by-step indexing-mechanism housing (34), which is configured as a flat plate, in such a manner that, in addition to the latching to the collar (41) of the cartridge (3), a second stronger latching takes place in the hand-held device housing (2).

5. Hand-held device according to one or more of the preceding claims, **characterized in that** the stronger latching in the hand-held device housing (2) can itself be released after removal of the cartridge (3) from the hand-held device housing (2).

6. Hand-held device according to claim 4, **characterized in that** the cartridge (3) is blocked against being pulled off by means of retaining fingers (55) which are formed pointing obliquely downwards in the hand-held device housing (2).

## Revendications

1. Dispositif manuel (1) de délivrance fractionnée de substances pulvérisables, en particulier des médicaments à inhaler, ayant une cartouche (3) qui, par appui, est déplaçable linéairement dans un boîtier (2) vers une position d'ouverture de délivrance laquelle cartouche repose avec son tube de soupape sur une partie de support (9) et un mécanisme d'indexage (11) se déplaçant lors de la course d'ouverture de la cartouche pour indexer et indiquer les actionnements de délivrance réalisés, lequel mécanisme d'indexation (11) est agencé dans un boîtier (34) centralement sous la paroi avant de la cartouche (3) du côté de son ouverture, en chevauchement du tube de soupape (5) de la cartouche et avec des éléments d'indexation de mécanisme pas-à-pas (S, 28, 29, 16, 17, 20, 15, 21, 22, 13, 26) agencés dans le boîtier (34), lesquels tournent autour d'axes s'étendant en direction longitudinale de la cartouche (3), dans lequel des doigts élastiques d'indexation pas-à-pas (28) se déforment vers une position transversale à la direction longitudinale et provoquent une rotation du mécanisme pas-à-pas, **caractérisé en ce que** le boîtier (34), y inclut son fond (42) et le mécanisme pas-à-pas (11, 12), est dans son ensemble - à l'exception d'une étoile à doigts d'indexation pas-à-pas (S) en appui sur la partie de support (9) - déplaçable vers le bas du fait de l'avancée de la cartouche relativement à la partie de support (9) et que le mouvement de déplacement fait tourner l'étoile à doigts d'indexation pas-à-pas (S).

2. Dispositif manuel selon la revendication 1, **caractérisé en ce que** les éléments d'indexation du mécanisme pas-pas comprennent une couronne dentée (16) laquelle comprend au moins une fente s'étendant de manière inclinée vers le haut depuis le bord inférieur pour être engagée par un tenon de guidage (33) de l'étoile à doigts d'indexation pas-à-pas (S).

3. Dispositif manuel selon une ou plusieurs des revendications précédentes, **caractérisé en ce que** le boîtier (34) du mécanisme pas-à-pas réalisé sous forme d'assiette plate comprend un trou central (38) pour le passage du tube de soupape (5), lequel trou (38) s'élargit à l'extrémité du côté de la cartouche pour la pénétration d'un col (41) en saillie sur le côté de l'ouverture de la cartouche, derrière lequel se produit un accrochage élastique entre boîtier (34) du mécanisme pas-à-pas et cartouche (3).

4. Dispositif manuel selon une ou plusieurs des revendications précédentes, **caractérisé par** un double accrochage du boîtier (34) du mécanisme pas-à-pas sous forme d'assiette plate de façon à ce qu'à côté de l'accrochage au col (41) de la cartouche (3) se produit un deuxième accrochage plus fort dans le boîtier (2) du dispositif manuel.

5. Dispositif manuel selon la revendication 4, **caractérisé en ce que** l'accrochage plus fort dans le boîtier du dispositif manuel (2) peut être défait après extraction de la cartouche (3) hors du boîtier (2) du dispositif manuel.

6. Dispositif manuel selon une ou plusieurs des revendications précédentes, **caractérisé en ce que** la cartouche (3) est bloquée contre l'extraction par des doigts de retenue (55) qui sont agencés dans le boîtier (2) du dispositif manuel en s'étendant de manière inclinée vers le bas.
